# DEMANDE DE BREVET EUROPEEN

(11) **EP 1 064 926 A1**
(43) Date de publication de la demande: **03.01.2001**
(21) Numéro de dépôt: 00401423.9
(22) Date de dépôt: 23.05.2000
(51) Int. Cl.: A61K 7/48, A61K 7/00, A61K 7/02

(54) **Composition cosmétique multiphases à base d'une huile fluorée volatile**

(30) Priorité: 30.06.1999 FR 9908371
(71) Demandeur: L'OREAL, 75008 Paris (FR)
(72) Inventeur: Lorant, Raluca, 94320 Thiais (FR)
(74) Mandataire: Dodin, Catherine

(57) **Abrégé**

L'invention concerne une composition cosmétique multiphases, se présentant sous forme de phases fluides et distinctes dont l'une contient au moins une huile fluorée volatile de densité particulière.
De préférence la composition se présente sous forme d'un biphase ou d'un tri-phase, la phase contenant l'huile fluorée volatile étant la phase inférieure.

Utilisation en cosmétique pour le démaquillage et les soins corporels.

## Description

La présente invention a pour objet de nouvelles compositions cosmétiques multiphases se présentant sous forme de phases fluides et distinctes, mutuellement insolubles, l'une des phases contenant au moins une huile fluorée volatile ayant une densité particulière.

Plus particulièrement, la présente invention a pour objet des compositions biphase ou triphases pour le démaquillage ou les soins corporels.
Lorsque les compositions selon l'invention sont du type biphase, la phase inférieure est essentiellement constituée d'au moins l'huile fluorée volatile, et la phase supérieure est soit aqueuse soit huileuse.
Par contre, lorsque les compositions selon l'invention sont du type triphases, la phase inférieure est également telle que définie ci-dessus, mais la phase intermédiaire peut être soit une phase aqueuse, soit une phase huileuse et la phase supérieure est une phase huileuse, non miscible, de densité inférieure à la phase sous-jacente.
Des lotions multiphases pour le démaquillage et les soins de la peau ont déjà été décrites et l'on peut à cet égard citer notamment le brevet FR-2 638 636 qui décrit une composition cosmétique pour le démaquillage des yeux du type biphase comportant une phase aqueuse inférieure et une phase huileuse supérieure, la phase aqueuse contenant un agent tensioactif.
Si ce type de composition permet une bonne élimination des produits de maquillage, elle présente néanmoins l'inconvénient de contenir un agent tensioactif susceptible de provoquer certaines irritations. Par ailleurs il n'est pas possible d'obtenir un déphasage rapide dans la mesure où, après agitation, les phases ont tendance à rester à l'état émulsionné.
L'obtention d'un déphasage rapide étant souhaitable pour diverses raisons, notamment d'un point de vue esthétique, il a été proposé dans le brevet FR-2 699 404 d'utiliser en tant qu'agent de déphasage du chlorure de benzalkonium.
Il a également été proposé dans le brevet FR-2 680 686 des lotions triphases comprenant une phase huileuse supérieure, une phase siliconée intermédiaire et une phase aqueuse inférieure.
Ces compositions, si elles possèdent de bonnes propriétés cosmétiques notamment de démaquillage ont cependant l'inconvénient d'une perte d'efficacité dans le temps, dans la mesure où les huiles siliconées de la phase intermédiaire sont volatiles et partiellement solubles dans la phase huileuse supérieure. Il en résulte qu'à l'usage, la phase siliconée intermédiaire a tendance à diminuer par rapport aux deux autres phases de la composition et à perdre ainsi de son efficacité dans le temps.

Après diverses études, on a maintenant constaté qu'il était possible de remédier aux inconvénients des compositions multiphases de l'état de la technique en utilisant certaines huiles fluorées volatiles de densité supérieure à 1 qui constituent de préférence la phase inférieure de compositions multiphases.
En effet on a observé de façon inattendue que l'utilisation d'huiles fluorées volatiles dans des compositions de ce type permettait d'apporter des propriétés démaquillantes particulièrement performantes tout en conférant un effet de fraîcheur immédiate dès l'application.

Par ailleurs, l'huile fluorée volatile étant de préférence présente dans la phase inférieure, il ne peut de ce fait se produire de pertes par volatilité, cette phase se trouvant en effet être protégée par la phase immédiatement supérieure qui, comme on l'a décrit ci-dessus, peut être soit une phase aqueuse, soit une phase huileuse non miscible.
Il s'est révélé en outre que les compositions biphase et triphases selon l'invention, après agitation et utilisation, avaient une vitesse de déphasage particulièrement rapide ce qui par conséquent réduit considérablement les pertes éventuelles par volatilité de la phase inférieure.
Il convient enfin de remarquer que selon la présente invention les compositions biphase et triphases sont de préférence essentiellement exemptes d'agent tensioactif, ce qui bien entendu présente par ailleurs un intérêt supplémentaire dans la mesure où, comme ceci a été précisé ci-dessus, l'emploi d'un agent tensioactif est susceptible de provoquer des phénomènes d'irritation des yeux et de la peau.

La présente invention a donc pour objet une composition cosmétique multiphases, se présentant sous forme de phases fluides et distinctes, caractérisée par le fait que l'une des phases de ladite composition contient au moins une huile fluorée volatile de densité supérieure à 1.
Un autre objet de l'invention est l'utilisation d'une telle composition pour le démaquillage ou le soin des matières kératiniques, notamment de la peau du corps et/ou du visage, des lèvres et/ou des fibres kératiniques (ongles, cils, sourcils, cheveux).

Selon l'invention, la densité de l'huile fluorée volatile est généralement supérieure à environ 1,1 et de préférence supérieure à 1,2.
Par l'expression "huile fluorée volatile", on doit entendre une huile ayant, à 25°C, une pression de vapeur saturante au moins égale à 50 Pa.
Selon une forme de réalisation préférée, l'huile fluorée volatile de densité supérieure à environ 1 constitue essentiellement la phase inférieure de la composition selon l'invention.
Parmi les huiles fluorées volatiles répondant aux critères ci-dessus de densité, de tension et de vapeur, on peut notamment citer les composés fluorés suivants, seuls ou en mélange :
1°) les perfluorocycloalkyles répondant à la formule (I) suivante : 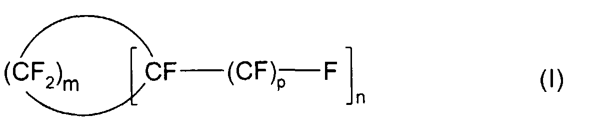 dans laquelle :
   m est 4 ou 5
   n est 1 ou 2, et
   p est 1, 2 ou 3
   sous réserve que lorsque n = 2, les groupements ne sont pas nécessairement en alpha, l'un par rapport à l'autre ;
2°) les perfluoroalcanes répondant à la formule (II) suivante :

   CF₃-(CF₂)ₘ-CF₂X (II)

   dans laquelle :
   m est 2 à 8, et
   X représente Br ou F ;
3°) les fluoroalkyles ou hétérofluoroalkyles répondant à la formule (III) suivante :

   CH₃-(CH₂)ₙ-[Z]ₜ-(CF₂)ₘ-CF₃ (III)

   dans laquelle :
   t est 0 ou 1,
   n est 0, 1, 2 ou 3,
   m est 2, 3, 4 ou 5, et
   Z est O, S ou NR, R étant un hydrogène, un radical -(CH₂)ₘ-CH₃, ou -(CF₂)ₘ-CF₃; et
4°) les dérivés de perfluoromorpholine répondant à la formule (IV) suivante : 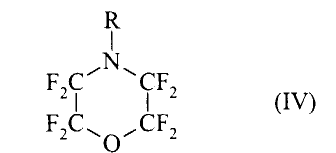 dans laquelle R est un radical perfluoroalkyle en C₁-C₄.

Parmi les perfluorocycloalkyles de formule (I), on peut notamment citer le perfluorométhylcyclopentane et le perfluoro-1,3-diméthylcyclohexane vendus sous les dénominations de "Flutec PC1®" et "Flutec PC3®" par la Société BNFL FLUOROCHEMICALS Ltd., qui ont des densités respectives de 1,26 et 1,82, ainsi que le perfluoro-1,2-diméthylcyclobutane.
Parmi les perfluoroalcanes de formule (II), on peut citer, entre autres, le dodécafluoropentane et le tetradécafluorohexane, vendus sous les dénominations de "PF 5050®" et "PF 5060®" par la Société 3M et qui ont des densités respectives de 1,63 et 1,68 ou encore le bromoperfluorooctyle vendu sous la dénomination de "FORALKYL®" par la Société ATOCHEM de densité 1,95.
Parmi les composés fluorés de formule (III), on peut citer par exemple, le nonafluorométhoxybutane vendu sous la dénomination de "MSX 4518®" par la Société 3M, de densité 1,53 et le nonafluoroéthoxyisobutane de densité 1,43.
Enfin, parmi les dérivés de perfluoromorpholine de formule (IV), on peut citer par exemple la 4-trifluorométhyl perfluoromorpholine vendue par la Société 3M sous la dénomination "PF 5052®" de densité égale à 1,7.

Les composés fluorés tels que décrits ci-dessus sont par ailleurs caractérisés par un point d'ébullition généralement compris entre 25 et 65°C.

La proportion en huile fluorée volatile dans la phase inférieure est généralement comprise entre 10 et 100 % par rapport au poids total de ladite phase, de préférence entre 15 et 90 %, et plus particulièrement entre 25 et 75 %.

En effet, selon l'invention, la phase inférieure à base d'une huile fluorée volatile peut éventuellement se présenter sous forme d'un mélange homogène avec au moins une huile non fluorée miscible, sous réserve toutefois qu'elle ne modifie pas sensiblement la densité de la phase inférieure, celle-ci devant être comme précédemment mentionnée, supérieure à 1.

Parmi les huiles non fluorées miscibles avec l'huile fluorée volatile, on peut mentionner les polysiloxanes notamment les PDMS en particulier volatils tels que le cyclopentasiloxane et le cyclohexasiloxane, les huiles hydrocarbonées volatiles et notamment les isoparaffines en C₁₁-C₁₃ ainsi que l'isododécane.

Selon un premier mode de réalisation de l'invention, les compositions se présentent sous forme d'un biphasé, la phase inférieure comprenant l'huile fluorée volatile de densité supérieure à 1, soit seule, soit en mélange avec une huile miscible telle définie ci-dessus et la phase supérieure, de densité inférieure ou égale à 1, étant constituée soit d'une phase aqueuse, soit d'une phase huileuse non miscible.
Lorsque la phase supérieure est une phase aqueuse, celle-ci est constituée généralement par de l'eau déminéralisée stérile ou éventuellement par une eau florale telle que de l'eau de rose, de l'eau de bleuet, de l'eau de camomille ou de l'eau de tilleul.
Lorsque la phase supérieure est une phase huileuse, celle-ci peut être constituée d'au moins une huile non fluorée de densité inférieure à 1, non miscible avec l'huile fluorée volatile de la phase inférieure seule ou sous forme d'un mélange tel que défini ci-dessus.

Parmi les huiles susceptibles de constituer la phase supérieure de ce type de biphase on peut citer certaines huiles minérales, végétales ou synthétiques. Parmi les huiles minérales, on peut notamment citer l'huile de vaseline (densité 0,84) ; parmi les huiles végétales celles contenant des triglycérides d'acides gras telles que par exemple l'huile d'amande d'abricot, l'huile de soja et l'huile d'amande douce, et parmi les huiles synthétiques, on peut citer le polyisobutène hydrogéné (densité 0,81), le di-octylcyclohéxane (densité 0,81) et les esters d'acides gras tels que le palmitate d'isopropyle (densité 0,85), le palmitate d'octyle et l'adipate de dioctyle.
Selon cette première forme de réalisation de l'invention chacune des deux phases est présente en une proportion comprise de préférence entre environ 40 et 60 % v/v.

Selon une deuxième forme de réalisation des compositions selon l'invention, celles-ci se présentent sous forme d'un triphase.
Selon une première variante de ce mode de réalisation, le triphase comporte une phase inférieure comprenant une huile fluorée volatile de densité supérieure à 1, une phase intermédiaire aqueuse et une phase huileuse supérieure non miscible avec la phase inférieure, et ayant une densité inférieure à la phase aqueuse.
Selon une deuxième variante, le triphase comporte une phase inférieure comprenant l'huile fluorée volatile de densité supérieure à 1, une phase intermédiaire huileuse et une phase huileuse supérieure, lesdites phases étant de densité distincte et non miscibles entre elles.
Les huiles susceptibles de constituer la phase intermédiaire, non miscible avec la phase inférieure contenant l'huile fluorée volatile et avec la phase huileuse supérieure, peuvent être aisément sélectionnées par de simples essais de routine parmi les huiles de densité comprise entre celle de l'huile de la phase supérieure et celle de l'huile de la phase inférieure.
Selon cette forme de réalisation la phase huileuse supérieure est essentiellement constituée d'au moins une huile telle que décrite ci-dessus pour les compositions se présentant sous forme d'un biphase et dont la phase supérieure est une phase huileuse.
Selon cette forme particulière de réalisation de l'invention, chacune des trois phases est présente en une proportion comprise de préférence entre environ 30 et 40 % v/v.

Bien que la présence d'un agent tensioactif ne soit pas nécessaire en vue d'obtenir de bons résultats, il peut être utile, dans certains cas, d'en introduire une faible proportion dans au moins l'une des phases.
Les tensioactifs qui peuvent être utilisés sont bien connus en cosmétique et ont été notamment décrits dans le brevet FR-2 697 404.

Les compositions sous forme de lotions biphase ou triphases peuvent comprendre certains principes actifs cosmétiques solubilisés dans l'une des phases des compositions, ceci afin de leur conférer certaines propriétés particulières par exemple une activité hydratante, anti-solaire, émolliente, anti-vieillissement, amincissante, etc.

Les compositions selon l'invention peuvent notamment se présenter sous la forme de compositions pour le démaquillage ou le soin des matières kératiniques, notamment de la peau du corps et/ou du visage, des lèvres et/ou des fibres kératiniques (ongles, cils, sourcils, cheveux).

Les divers principes actifs hydrophiles ou lipophiles susceptibles d'être introduits dans l'une au moins des phases des compositions selon l'invention ont été décrits dans le brevet FR-2 680 686.

L'intérêt des compositions sous forme de biphase ou de triphases est de pouvoir introduire dans des phases différentes des composés actifs qui seraient autrement incompatibles entre eux.
En plus de principes actifs éventuellement présents dans au moins l'une des phases, celles-ci peuvent également contenir, en fonction de leur solubilité, des ingrédients cosmétiques conventionnels tels que des colorants, des parfums ou des agents conservateurs.
Bien qu'il ait été plus particulièrement fait référence à l'usage des compositions selon l'invention pour le démaquillage des yeux ou du visage, il va de soi que celles-ci peuvent également être utilisées en vue d'autres applications telles que par exemple des produits anti-solaires, des produits auto-bronzants, des produits de maquillage ou des produits pour le traitement ou les soins de la peau et des cheveux.
On va maintenant donner à titre d'illustration, plusieurs exemples de compositions cosmétiques selon l'invention.

### EXEMPLE 1 : Lotion biphase hydratante

Cette lotion, conditionnée dans un flacon, est constituée des deux phases suivantes :

### A - Phase supérieure (phase aqueuse)

- Glycérol 5,0 %
- Conservateurs 0,3 %
- Eau 44,7 %

### B - Phase inférieure

- 4-trifluorométhyl perfluoromorpholine 35,0 %
- Cyclopentasiloxane 15,0 %

Lors de l'utilisation, on procède à l'agitation du flacon, contenant les deux phases, ce qui provoque une émulsion temporaire. Par application sur le visage, à l'aide d'un coton par exemple, on observe un effet adoucissant et hydratant ainsi que des propriétés de fraîcheur. Par ailleurs, après repos du flacon, on constate un déphasage de l'émulsion en quelques minutes.

### EXEMPLE 2 : Lotion biphase démaquillante

Une lotion de démaquillage biphase contenant les deux phases suivantes est conditionnée dans un flacon :

### A - Phase supérieure (phase huileuse)

- Palmitate d'isopropyle 50 %

### B - Phase inférieure

- Nonafluorométhoxybutane 25 %
- Cyclopentasiloxane 25 %

Par agitation, cette lotion anhydre biphase conduit à une émulsion qui permet un excellent démaquillage de produits de maquillage de longue tenue ou "waterproof". La présence de l'huile fluorée volatile facilite l'élimination du maquillage tout en conférant un effet de fraîcheur. Un déphasage se produit quelques minutes après l'agitation.

### EXEMPLE 3 : Lotion biphase corporelle

On conditionne dans un flacon les deux phases suivantes :

### A - Phase supérieure (phase huileuse)

- Huile d'amande d'abricot 25 %
- Isoparaffine hydrogénée 25 %

### B - Phase inférieure

- 4-trifluorométhyl perfluoromorpholine 25 %
- Cyclohexasiloxane 25 %

Après agitation, cette lotion est appliquée sur le corps et confère des propriétés tonifiantes et hydratantes. Au cours de l'utilisation dans le temps on ne dénote aucune diminution du rapport en volume entre la phase inférieure et la phase supérieure de la lotion.

### EXEMPLE 4 : Lotion triphase pour soins nutritifs

On obtient une lotion triphase en conditionnant dans un flacon les trois phases suivantes :

### A - Phase supérieure (phase huileuse)

- Huile d'amande d'abricot 33,33 %

### B - Phase intermédiaire (phase aqueuse)

- Glycérol 5,00 %
- Conservateur 0,40 %
- Eau 28,03 %

### C - Phase inférieure

- Nonafluorométhoxybutane 23,33 %
- Cyclohexasiloxane 10,00 %

Après agitation, la lotion est appliquée sur le visage apportant des propriétés cosmétiques d'hydratation et de fraîcheur à la peau.
Au cours de l'utilisation dans le temps, on ne constate aucune variation dans le rapport en volume des trois phases de la lotion.

### EXEMPLE 5 : Lotion biphase démaquillante

Une lotion de démaquillage biphase contenant les deux phases suivantes est conditionnée dans un flacon :

### A - Phase supérieure (phase huileuse)

- Palmitate d'isopropyle 25 %
- Adipate de dioctyle 25 %

### B - Phase inférieure

- Nonafluorométhoxybutane 20 %
- Tétradécafluorohexane 10 %
- Cyclopentasiloxane 20 %

Au moment de l'emploi on agite le flacon et on applique la lotion à l'aide d'un coton sur les parties de la peau à démaquiller. On constate une bonne élimination du maquillage et une action tonifiante et hydratante de la lotion. Après quelques minutes de repos, on observe une nette séparation des phases.

## Revendications

1. Composition cosmétique multiphases, se présentant sous forme de phases fluides et distinctes, caractérisée par le fait que l'une des phases de ladite composition contient au moins une huile fluorée volatile de densité supérieure à 1.

2. Composition selon la revendication 1, caractérisée par le fait que l'huile fluorée volatile a une densité supérieure à 1,1, de préférence 1,2.

3. Composition selon l'une des revendication précédentes, caractérisée par le fait que la phase contenant l'huile fluorée volatile constitue la phase inférieure de ladite composition.

4. Composition selon l'une quelconque des revendications précédentes, caractérisée par le fait que l'huile fluorée volatile est choisie parmi, seuls ou en mélange :
1°) les perfluorocycloalkyles répondant à la formule (I) suivante : 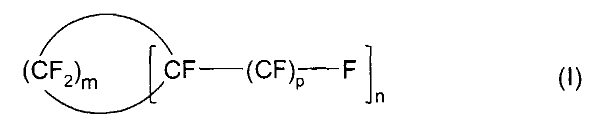 dans laquelle :
m est 4 ou 5; n est 1 ou 2, et p est 1, 2 ou 3, sous réserve que lorsque n = 2, les groupements ne sont pas nécessairement en alpha l'un par rapport à l'autre.
2°) les perfluoroalcanes répondant à la formule (II) suivante :
CF₃-(CF₂)ₘ-CF₂X (II)
dans laquelle :
m est 2 à 8 et X représente Br ou F;
3°) les fluoroalkyles ou hétérofluoroalkyles répondant à la formule (III) suivante :
CH₃-(CH₂)ₙ-[Z]ₜ-(CF₂)ₘ-CF₃ (III)
dans laquelle :
t est 0 ou 1; n est 0, 1, 2 ou 3; m est 2, 3, 4 ou 5, et
Z est O, S ou NR, R étant un hydrogène, un radical-(CH₂)ₘ-CH₃ ou -(CF₂)ₘ-CF₃;
4°) les dérivés de perfluoromorpholine répondant à la formule (IV) suivante : 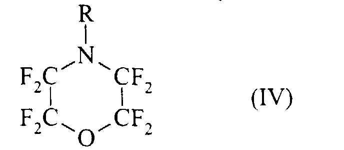 dans laquelle R est un radical perfluoroalkyle en C₁-C₄.

5. Composition selon l'une quelconque des revendications précédentes, caractérisée par le fait que l'huile fluorée volatile est choisie dans le groupe constitué par le perfluorométhylcyclopentane, le perfluoro-1,3 diméthylcyclohexane, le perfluoro-1,2-diméthylcyclobutane, le dodécafluoropentane, le tétradécafluorohexane, le bromoperfluorooctyle, le nonafluorométhoxybutane le nonafluoroéthoxyisobutane et la 4-trifluorométhyl perfluoromorpholine.

6. Composition selon l'une quelconque des revendications précédentes, caractérisée par le fait que la proportion en huile fluorée volatile dans la phase inférieure est comprise entre 10 et 100 % par rapport au poids total de ladite phase, de préférence entre 15 et 90 %, et plus particulièrement entre 25 et 75 %.

7. Composition selon l'une quelconque des revendications précédentes, caractérisée par le fait que ladite phase inférieure comprend en outre au moins une huile non fluorée miscible avec l'huile fluorée volatile.

8. Composition selon la revendication 7, caractérisée par le fait que l'huile non fluorée miscible est choisie parmi les polysiloxanes, notamment les PDMS en particulier volatils tels que le cyclopentasiloxane et le cyclohexasiloxane, et les huiles hydrocarbonées volatiles, notamment les isoparaffines en C₁₁-C₁₃ et l'isododécane.

9. Composition selon l'une quelconque des revendications précédentes, caractérisée par le fait qu'elle se présente sous forme d'un biphase, la phase inférieure comprenant l'huile fluorée volatile de densité supérieure à 1 et la phase supérieure étant constituée soit d'une phase aqueuse, soit d'une phase huileuse non miscible.

10. Composition selon l'une quelconque des revendications 1 à 8, caractérisée par le fait qu'elle se présente sous forme d'un triphase, comportant une phase inférieure comprenant l'huile fluorée volatile de densité supérieure à 1, une phase intermédiaire aqueuse et une phase huileuse supérieure, non miscible avec la phase inférieure, et de densité inférieure à la phase aqueuse.

11. Composition selon l'une quelconque des revendications 1 à 8, caractérisée par le fait qu'elle se présente sous forme d'un triphase, comportant une phase inférieure comprenant l'huile fluorée volatile de densité supérieure à 1, une phase intermédiaire huileuse et une phase supérieure huileuse, lesdites phases étant de densité distincte et non miscibles entre elles.

12. Composition selon l'une des revendications précédentes, se présentant sous la forme de compositions pour le démaquillage ou le soin des matières kératiniques, notamment de la peau du corps et/ou du visage, des lèvres et/ou des fibres kératiniques (ongles, cils, sourcils, cheveux).

13. Utilisation d'une composition selon l'une des revendications précédentes pour le démaquillage ou le soin des matières kératiniques, notamment de la peau du corps et/ou du visage, des lèvres et/ou des fibres kératiniques (ongles, cils, sourcils, cheveux).
